# EUROPEAN PATENT SPECIFICATION

(11) **EP 1 485 090 B1**
(45) Date of publication and mention of the grant of the patent: **10.10.2007**
(21) Application number: 03720323.9
(22) Date of filing: 07.03.2003
(51) Int. Cl.: A61K 31/425

(54) **COMBINATIONS COMPRISING AN EPOTHILONE DERIVATIVE AND AN IMIDAZOTETRAZINONE**
KOMBINATIONSPRÄPARAT AUS EPOTHILONE DERIVATE UND IMIDAZOTETRAZINONE
MELANGES COMPRENANT UN DERIVE D'EPOTHILONE ET UNE IMIDAZOTETRAZINONE

(30) Priority: 08.03.2002 US 362738 P
(43) Date of publication of application: 15.12.2004
(60) Divisional application: 07110769.2
(73) Proprietor: Novartis AG, 4056 Basel (CH); Novartis Pharma GmbH, 1230 Wien (AT); THE UNIVERSITY OF NEWCASTLE UPON TYNE, Newcastle Upon Tyne NE1 7RU (GB)
(72) Inventor: ANDERSON, Judith, Rose, Denville, NJ 07834 (US); ROTHERMEL, John, David, Randolph, NJ 07869 (US); MC SHEEHY, Paul, M.J., 79540 Loerrach-Stetten (DE); BODDY, Alan, Blaydon, Tyne and Wear, NE21 A4Y (GB)
(74) Representative: de Weerd, Petrus G.W.
(86) International application number: PCT/EP2003/002364
(87) International publication number: WO 2003/075899

(56) References cited:
- US-B1- 6 262 094
- WOLFF ET AL: "Epothilone A induces apoptosis in neuroblastoma cells with multiple mechanisms of drug resistance" INTERNATIONAL JOURNAL OF ONCOLOGY, EDITORIAL ACADEMY OF THE INTERNATIONAL JOURNAL OF ONCOLOGY,, GR, vol. 11, no. 1, July 1997 (1997-07), pages 123-126, XP002111280 ISSN: 1019-6439
- SEPP-LORENZINO, L. ET AL: "The microtubule-stabilizing agents epothilones A and B and their desoxy-derivatives induce mitotic arrest and apoptosis in human prostate cancer cells" PROSTATE CANCER AND PROSTATIC DISEASES (1999), 2(1), 41-52 , 1999, XP000917730

## Description

The invention relates to a pharmaceutical combination which comprises (a) an imidazotetrazinone and (b) an epothilone derivative of formula I and optionally at least one pharmaceutically acceptable carrier for simultaneous, separate or sequential use, in particular for the treatment of a proliferative disease, especially a tumor disease; a pharmaceutical composition comprising such a combination; the use of such a combination for the preparation of a medicament for the treatment of a proliferative disease; and a commercial package or product comprising such a combination as a combined preparation for simultaneous, separate or sequential use.

The microtubule-stabilizing effect of epothilones was first described by Bollag et al., Cancer Research 1995, 55, 2325-33. A suitable treatment schedule, comprising epothilone dosing, of different types of tumors, especially tumors refractory to the treatment by other chemotherapeutics, in particular refractory to the treatment by taxanes, like TAXOL^{™}, is described in WO 99/43320. Estramustine, an estradiol nitrogen mustard conjugate, exerts anti-tumor effects binding with β-tubulin and microtubule-associated proteins (Scholz et al., Proc. Am. Soc. Clin. Oncol. 1998, 17, 342a).

US 6,262,094 discloses C-21 modified epothilones in conjunction with anticancer and cytotoxic agents.

Surprisingly, it has been found that the anti-proliferative effect on tumors of a combination which comprises an imidazotetrazinone and an epothilone is greater than the maximum effect that can be achieved with either type of ingredient alone.

The present invention pertains to a combination, such as a combined preparation or a pharmaceutical composition, which comprises (a) an alkylating agent and (b) an epothilone derivative of formula I 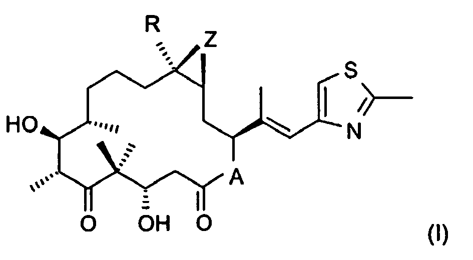 wherein A represents O or NR_{N}, wherein R_{N} is hydrogen or lower alkyl, R is hydrogen or lower alkyl, and Z is O or a bond, in which the active ingredients (a) and (b) are present in each case in free form or in the form of a pharmaceutically acceptable salt and optionally at least one pharmaceutically acceptable carrier; for simultaneous, separate or sequential use, in particular for the treatment of a proliferative disease, especially a solid tumor disease.

Unless stated otherwise, in the present disclosure organic radicals and compounds designated "lower" contain not more than 7, preferably not more than 4, carbon atoms.

The term "a combined preparation", as used herein defines especially a "kit of parts" in the sense that the combination partners (a) and (b) as defined above can be dosed independently of each other or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, a combined therapeutic effect in a non-effective dosage of one or both of the combination partners (a) and (b), and very preferably a strong synergism of the combination partners (a) and (b).

The term "treatment" comprises the administration of the combination partners to a warm-blooded animal in need of such treatment with the aim to have an effect on the delay of progression of a disease.

The term "delay of progression" as used herein means that the tumor growth or generally, the disease progression is at least slowed down or hampered by the treatment and that patients exhibit higher survival rates than patients not being treated or being treated with the monotherapy.

The term "proliferative disease" includes but is not restricted to tumors and psoriasis.

The term "tumor disease" means any neoplastic proliferative disorder e.g. solid tumor diseases or liquid tumor diseases.

The term "a solid tumor disease" especially means ovarian cancer, cancer of the colon and generally the gastrointestinal tract, cervix cancer, lung cancer, e.g. small-cell lung cancer and non-small-cell lung cancer, head and neck cancer, bladder cancer, cancer of the prostate or Kaposi's sarcoma. The combinations disclosed herein are also useful for the treatment of leukemia.

"Imidazotetrazinones" include but are not restricted to temozolomide and mitozolomide.

The structure of the active agents cited may be taken from the actual edition of the standard compendium "The Merck Index" or from databases, e.g. Patents International (e.g. IMS World Publications). The corresponding content thereof is hereby incorporated by reference. Any person skilled in the art is fully enable, based on these references, to manufacture and test the pharmaceutical indications and properties in standard test models, both *in vitro* and *in vivo.*

Epothilone derivatives of formula I wherein A represents O or NR_{N}, wherein R_{N} is hydrogen or lower alkyl, R is hydrogen or lower alkyl and Z is O or a bond, and methods for the preparation of such epothilone derivatives are in particular generically and specifically disclosed in the patents and patent applications WO 93/10121, US 6,194,181, WO 98/25929, WO 98/08849, WO 99/43653, WO 98/22461 and WO 00/31247. Comprised are likewise the corresponding stereoisomers as well as the corresponding crystal modifications, e.g. solvates and polymorphs, which are disclosed therein. Epothilone derivatives of formula I, especially epothilone B, can be administered as part of pharmaceutical compositions which are disclosed in WO 99/39694.

A compound of formula I wherein A represents O, R is hydrogen and Z is O is known as epothilone A; a compound of formula I wherein A represents O, R is methyl and Z is O is known as epothilone B; a compound of formula I wherein A represents O, R is hydrogen and Z is a bond is known as epothilone C; a compound of formula I wherein A represents O, R is methyl and Z is a bond is known as epothilone D.

The transformation of epothilone B to the corresponding lactam is disclosed in Scheme 21 (page 31, 32) and Example 3 of WO 99/02514 (pages 48 - 50). The transformation of a compound of formula I, which is different from epothilone B into the corresponding lactam can be accomplished analogously. Corresponding epothilone derivatives of formula I wherein R_{N} is lower alkyl can be prepared by methods known in the art such as a reductive alkylation reaction starting from the epothilone derivative wherein R_{N} is hydrogen.

The term "temozolomide" means a compound as described in US 5,260,291. The synthesis of temozolomide is well known e.g., Wang et al., J. Org. Chem. 1997, 62, 7288-7294. Temozolomide is commercially available e.g. under the trademark of TEMODAL^{™}, TEMODAR^{™}, or TEMOXOL^{™} and can be administered, e.g., as described in US 5,942,247 or according to the package insert information.

The compounds used as combination partners (a) and (b) disclosed herein can be prepared and administered as described in the cited documents, respectively.

It will be understood that references to the combination partners (a) and (b) are meant to also include the pharmaceutically acceptable salts. If these combination partners (a) and (b) have, for example, at least one basic center, they can form acid addition salts, e.g. succinates. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization.

A combination which comprises (a) an imidazotetrazinone and (b) an epothilone derivative of formula I in which compound A represents O or NR_{N}, wherein R_{N} is hydrogen or lower alkyl, R is hydrogen or lower alkyl, and Z is O or a bond, in which the active ingredients are present in each case in free form or in the form of a pharmaceutically acceptable salt and optionally at least one pharmaceutically acceptable carrier, will be referred to hereinafter as a COMBINATION OF THE INVENTION.

The COMBINATIONS OF THE INVENTION inhibit the growth of solid tumors, but also liquid tumors. Furthermore, the COMBINATIONS OF THE INVENTION exhibit beneficial effects in the treatment of diseases associated with deregulated angiogenesis. In one preferred embodiment of the invention, the proliferative disease to be treated with a COMBINATION OF THE INVENTION is prostate cancer, especially adenocarcinoma of the prostate gland, especially in patients that have undergone prior treatment with bilateral orchiectomy or luteinizing hormone-releasing hormone agonist with evidence of treatment failure.

All the more surprising is the experimental finding that *in vivo* the administration of a COMBINATION OF THE INVENTION compared to a monotherapy applying only one of the pharmaceutically active ingredients used in the COMBINATION OF THE INVENTION results not only in a more beneficial, especially synergistic, e.g. anti-proliferative effect, e.g. with regard to the delay of progression of a proliferative disease or with regard to a change in tumor volume, but also in further surprising beneficial effects, e.g. less side-effects and a decreased mortality and morbidity. Furthermore, depending on the tumor type and the particular combination used a decrease of the tumor volume can be obtained when using a COMBINATION OF THE INVENTION in cases in which by monotherapy no decrease of the tumor volume can be achieved. The COMBINATIONS OF THE INVENTION are also suitable to prevent the metastatic spread of tumors and the growth or development of micro metastases.

A further benefit is that lower doses of the active ingredients of the COMBINATION OF THE INVENTION can be used, for example, that the dosages need not only often be smaller but are also applied less frequently, or can be used in order to diminish the incidence of side-effects like, e.g., diarrhea or nausea observed with one of the combination partners alone. This is in accordance with the desires and requirements of the patients to be treated.

It can be shown by established test models that a COMBINATION OF THE INVENTION results in the beneficial effects described herein before. The person skilled in the pertinent art is fully enabled to select a relevant test model to prove such beneficial effects. The pharmacological activity of a COMBINATION OF THE INVENTION may, for example, be demonstrated in a clinical study or in a test procedure as essentially described hereinafter.

Suitable clinical studies are in particular randomized, double-blind, placebo-controlled, parallel studies in cancer patients with late stage disease. Such studies are, in particular, suitable to compare the effects of a monotherapy using the active ingredients and a therapy using a COMBINATION OF THE INVENTION, and to prove in particular the synergism of the active ingredients of the COMBINATIONS OF THE INVENTION. The primary endpoints in such studies can be the effect on pain scores, analgesic use, performance status, Quality of Life scores or time to progression of the disease. The evaluation of tumors by spiral computer-assisted tomography (CT) scan and magnetic resonance imaging (MRI) in regular time periods, e.g. every 8 weeks, is a suitable approach to determine the effect of the COMBINATION OF THE INVENTION. In a suitable study design, patients are, for example, receiving per treatment cycle of four weeks a fixed dosage ranging from about 10 to 16 mg/kg/day of estramustine given in a 3 or 4 divided dose for 3 days per weeks for two weeks followed by two weeks off in addition to 0.5, 1.0, 1.5, 2.0 or 2.5 mg/m² of body surface of a compound of formula I, e.g. epothilone B or a corresponding placebo wherein compound of formula I is administered as a 5 minute bolus infusion once a week for three weeks followed by one week of rest. Alternatively, the compound of formula I can be administered once every three weeks. The minimum duration of such a study should be about 8 weeks.

It is one objective of this invention to provide a pharmaceutical composition comprising a quantity, which is jointly therapeutically effective against a proliferative disease comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carriers, especially suitable for enteral or parenteral application. In one embodiment of the invention, one or more of the active ingredients are administered intravenously.

The novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partners of the COMBINATION OF THE INVENTION may be administered simultaneously or sequentially and in any order, and the components may be administered separately or as a fixed combination. For example, the method of delay of progression or treatment of a proliferative disease according to the invention may comprise (i) administration of the first combination partner in free or pharmaceutically acceptable salt form and (ii) administration of the second combination partner in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise.

In particular, if the warm-blooded animal is a human, compound of formula I is preferably administered at a single 5 to 7 minutes infusion, most preferably 5 minutes. The infusion can be administered every week for 6 weeks followed by 3 weeks off or every week for 3 weeks followed by one week off at a dose of 0.3 to 2.5 mg/m². Compound of formula I can also be administered at a single infusion every 3 weeks at a dose of 0.3 to 6.0 mg/m². Most preferably, the infusion is a bolus infusion.

In the compound of formula I preferably A represents O. R is hydrogen or, preferably, lower alkyl, e.g. ethyl or, most preferably, methyl. Z is preferably O or a bond, more preferably O.

In one preferred embodiment of the invention, the imidazotetrazinone is temozolamide.

The COMBINATION OF THE INVENTION can be a combined preparation or a pharmaceutical composition.

In one embodiment of the invention, the COMBINATION OF THE INVENTION is co-administrable with an anti-emetic agent. Furthermore, the treatment can comprise surgery, radiotherapy, cryotherapy and immunotherapy.

Furthermore, the present invention pertains to the use of a COMBINATION OF THE INVENTION for the preparation of a medicament for the treatment of a proliferative disease.

Additionally, the present invention pertains to the use of imidazotetrazinone in combination with an epothilone derivative of formula I in which compound A represents O or NR_{N}, wherein R_{N} is hydrogen or lower alkyl, R is hydrogen or lower alkyl, and Z is O or a bond, for the preparation of a medicament for the treatment of a proliferative disease.

Moreover, the present invention provides a commercial package comprising as active ingredients COMBINATION OF THE INVENTION, together with instructions for simultaneous, separate or sequential use thereof in the treatment of a proliferative disease.

### Example 1: (Not forming part of the invention)

Patients with histologically or cytologically confirmed advanced cancer (stage III or IV) i.e. mesurable lesion of al least 2 cm. are enrolled in the study for at least 9 months. For each 4 week cycle, estramustine is administered orally for 3 following days per week for 2 weeks followed by two weeks off at a fixed dosage of 420 mg 3 times a day on Day I, the morning dose of Day 2 is 420 mg followed by afternoon and evening doses of 280 mg and on Day 3 the patients receive 3 times 280 mg. The dose may be reduced by 30 % if toxicity occurs. Estramustine is taken one hour before or 2 hours after meal with water, calcium rich products have to be avoided. For each 4 week cycle, compound of formula I is administered as a single bolus infusion over 5 minutes every week for 3 weeks, followed by one week off. Five dose levels of compound of formula I are being tested 0.5, 1.0, 1.5, 2.0 or 2.5 mg/m² corresponding to 5 cohorts of patients. Dose adjustments of compound of formula I are made for any change in the body weight superior of 10 % of the original weight throughout the study. On weeks with estramustine administration, compound of formula I is administered as described above immediately after the morning oral dose of estramustine.

## Claims

1. A combination which comprises (a) an imidazotetrazinone and (b) an epothilone derivative of formula I 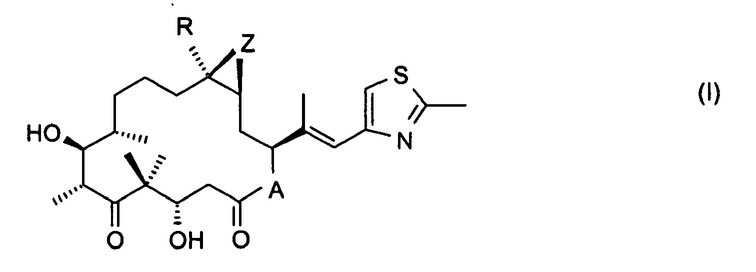 wherein A represents O or NR_{N}, wherein R_{N} is hydrogen or lower alkyl, R is hydrogen or lower alkyl, and Z is O or a bond, in which the active ingredients (a) and (b) are present in each case in free form or in the form of a pharmaceutically acceptable salt and optionally at least one pharmaceutically acceptable carrier; for simultaneous, separate or sequential use.

2. A combination according to claim 1 wherein the imidazotetrazinone is selected from temozolomide and mitozolomide.

3. A combination according to claim 1 or 2 comprising (b) an epothilone derivative of formula I wherein A represents O, R is lower alkyl or hydrogen and Z is O or a bond.

4. A combination according to any one of claims 1 to 3 wherein (a) is temozolomide.

5. A combination according to any one of claims 1 to 4 wherein compound (b) is an epothilone derivative of formula I wherein A represents O, R is methyl and Z is O.

6. Use of a combination according to any one of claims 1 to 5 for the preparation of a medicament in the treatment of a tumor disease.

7. A pharmaceutical composition comprising a quantity which is jointly therapeutically effective against a tumor disease of a pharmaceutical combination according to any one of claims 1 to 5 and at least one pharmaceutically acceptable carrier.

8. A commercial package comprising a combination according to any one of claims 1 to 5 together with instructions for simultaneous, separate or sequential use thereof in the treatment of a tumor disease.

## Patentansprüche

1. Kombination, umfassend (a) ein Imidazotetrazinon und (b) ein Epothilonderivat der Formel I 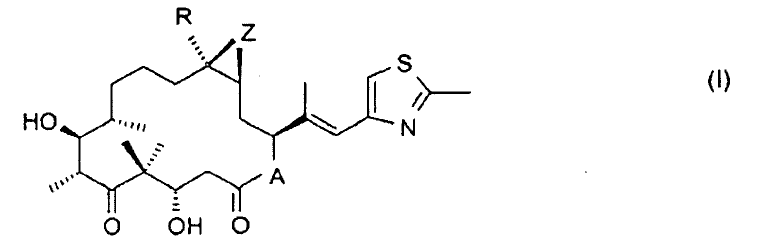 worin A für O oder NR_{N} steht, worin R_{N} für Wasserstoff oder Niederalkyl steht, R für Wasserstoff oder Niederalkyl steht und Z für O oder eine Bindung steht, worin die Wirkstoffe (a) und (b) in jedem Fall in freier Form oder in Form eines pharmazeutisch akzeptablen Salzes vorhanden sind, und optional wenigstens einen pharmazeutisch akzeptablen Träger, zur simultanen, separaten oder sequentiellen Anwendung.

2. Kombination nach Anspruch 1, worin das Imidazotetrazinon ausgewählt ist aus Temozolomid und Mitozolomid.

3. Kombination nach Anspruch 1 oder 2, umfassend (b) ein Epothilonderivat der Formel I, worin A für O steht, R für Niederalkyl oder Wasserstoff steht, und Z für O oder eine Bindung steht.

4. Kombination nach einem der Ansprüche 1 bis 3, worin (a) für Temozolomid steht.

5. Kombination nach einem der Ansprüche 1 bis 4, worin die Verbindung (b) ein Epothilonderivat der Formel I ist, worin A für O steht, R für Methyl steht und Z für O steht.

6. Verwendung einer Kombination nach einem der Ansprüche 1 bis 6 zur Herstellung eines Arzneimittels für die Behandlung einer Tumorkrankheit.

7. Pharmazeutische Zusammensetzung, umfassend eine Menge, die zusammen therapeutisch wirksam ist gegen eine Tumorkrankheit, einer pharmazeutischen Kombination nach einem der Ansprüche 1 bis 5 und wenigstens einen pharmazeutisch akzeptablen Träger.

8. Handelspackung, umfassend eine Kombination nach einem der Ansprüche 1 bis 5 zusammen mit Instruktionen zur simultanen, separaten oder sequentiellen Anwendung hiervon für die Behandlung einer Tumorkrankheit.

## Revendications

1. Combinaison qui comprend (a) une imidazotétrazinone et (b) un dérivé de l'épothilone de formule 1 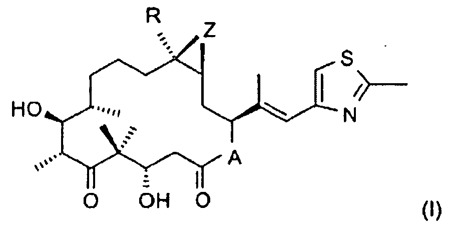 dans laquelle A représente O ou NR_{N}, où R_{N} est un atome d'hydrogène ou un groupe alkyle inférieur, R est un atome d'hydrogène ou un groupe alkyle inférieur et Z est O ou une liaison, où les principes actifs (a) et (b) sont présents dans chaque cas sous forme libre ou sous forme d'un sel acceptable d'un point de vue pharmaceutique, et éventuellement au moins un excipient acceptable d'un point de vue pharmaceutique ; en vue d'une utilisation simultanée, distincte ou séquentielle.

2. Combinaison selon la revendication 1, dans laquelle l'imidazotétrazinone est choisie parmi le témozolomide et le mitozolomide.

3. Combinaison selon la revendication 1 ou 2, comprenant (b) un dérivé de l'épothilone de formule I dans laquelle A représente O, R est un groupe alkyle inférieur ou un atome d'hydrogène, et Z est O ou une liaison.

4. Combinaison selon l'une quelconque des revendications 1 à 3, dans laquelle (a) est le témozolomide.

5. Combinaison selon l'une quelconque des revendications 1 à 4, dans laquelle le composé (b) est un dérivé de l'épothilone de formule I dans laquelle A représente O, R est le groupe méthyle et Z est O.

6. Utilisation d'une combinaison selon l'une quelconque des revendications 1 à 5 pour préparer un médicament destiné au traitement d'une maladie tumorale.

7. Composition pharmaceutique comprenant une quantité qui est conjointement efficace thérapeutiquement à l'encontre d'une maladie tumorale, d'une combinaison pharmaceutique selon l'une quelconque des revendications 1 à 5, et au moins un excipient acceptable d'un point de vue pharmaceutique.

8. Conditionnement commercial comprenant une combinaison selon l'une quelconque des revendications 1 à 5 en même temps que des instructions pour son utilisation simultanée, distincte ou séquentielle, pour le traitement d'une maladie tumorale.
